# EUROPÄISCHE PATENTSCHRIFT

(11) **EP 1 049 500 B1**
(45) Veröffentlichungstag und Bekanntmachung des Hinweises auf die Patenterteilung: **21.05.2003**
(21) Anmeldenummer: 98959819.8
(22) Anmeldetag: 31.10.1998
(51) Int. Cl.: A61M 3/02

(54) **VORRICHTUNG ZUR HARNBLASENSPÜLUNG**
DEVICE FOR WASHING-OUT THE BLADDER
DISPOSITIF DE LAVAGE VESICAL

(30) Priorität: 11.11.1997 DE 29720010 U
(43) Veröffentlichungstag der Anmeldung: 08.11.2000
(73) Patentinhaber: Bieber Biomedical Gmbh, 65719 Hofheim (DE)
(72) Erfinder: BIEBER, Rolf, D-60322 Frankfurt am Main (DE)
(74) Vertreter: Aue, Hans-Peter, Dipl-Ing.
(86) Internationale Anmeldenummer: EP9806900
(87) Internationale Veröffentlichungsnummer: WO99024089

(56) Entgegenhaltungen:
- US-A- 2 511 469
- US-A- 3 398 743
- US-A- 4 217 897
- US-A- 4 411 656
- US-A- 4 776 840
- US-A- 5 505 707

## Beschreibung

Die Erfindung betrifft eine Pumpe zur Spülung der Harnblase des Menschen über einen in die Harnblase gelegten Spülkatheter, mit einem ein definiertes Volumen aufweisenden kompressiblen Pumpkörper zur Erzeugung eines kurzzeitig erhöhten Spüldruckes zwischen Vorratsbehälter und Spülkatheter, einer an einen Vorratsbehälter für das Spülmedium angeschlossenen und in die Harnblase führenden Zufuhrleitung und einer aus der Harnblase herausführenden Abflussleitung.

Soweit es bei Blutungen der Harnblase einer Spülung zum Abführen von Blutkoageln aus der Harnblase bedarf, ist eine Vorrichtung entsprechend den vorgenannten Merkmalen durch Benutzung bekannt, mit der eine sogenannte Dauerspülung eingerichtet werden kann. Hierbei erfolgt von einem Vorratsbehälter für ein Spülmedium, beispielsweise Wasser, eine Zuleitung zu einem in die Harnblase gelegten Spülkatheter, wobei eine Forderung darin besteht, das System vom Vorratsbehälter für das Spülmedium bis zum Eintritt des Spülmediums in die Harnblase geschlossen zu halten, um den Zutritt von Bakterien und damit eine Infektion sicher zu verhindern.

Ein Problem ist im Rahmen derartiger Harnblasenspülung dadurch gegeben, dass es zum Lösen wie auch zur Abfuhr von Blutkoageln erforderlich werden kann, kurzzeitig den Spüldruck zu erhöhen. Hierzu ist es geläufig, bei der bekannten Vorrichtung die Abflußleitung kurzzeitig abzusperren, so dass aufgrund des anhaltenden Zulaufs von Spülmedium in die Harnblase der Spüldruck ansteigt; eine nachhaltige und medizinisch wirksame Erhöhung des Spüldrucks ist mit dieser Maßnahme jedoch nicht möglich. Alternativ ist der Einsatz einer sogenannten Blasenspritze bereits bekannt, für deren Gebrauch die Zufuhrleitung von dem Vorratsbehälter getrennt werden muß, um die Blasenspritze an der Zufuhrleitung ansetzen zu können. Hiermit ist nicht nur der Nachteil einer umständlichen und zeitaufwendigen Vorbereitung des Spülvorgangs verbunden, sondern auch der Nachteil der Unterbrechung des ansonsten geschlossenen Systems, so dass die Gefahr von Infekten real besteht.

Des weiteren ist aus der US-A-5,505,707 ein Schlauchsystem zum Fördern eines Spülmediums bekannt, dass jeweils mindestens zwei Ventile in der Zufuhrleitung aufweist. Zusätzlich ist im Schlauchsystem eine Schlauchklemme vorgesehen, die immer in Strömungsrichtung des Spülmediums nach dem Pumpkörper in der Zufuhrleitung angeordnet ist. Diese Schlauchklemme dient der Regulierung bzw. Absperrung des Spülmedienzuflusses hin zum intraoperativen Spülkatheter. Damit stellt die Schlauchklemme quasi eine drittes Ventil dar. Das erste Ventil verhindert eine Spülmedienströmung vom Pumpkörper in den Vorratsbehälter zurück, wenn der Pumpkörper zusammengedrückt wird. Das zweite Ventil ermöglicht das Strömen des Spülmediums vom Pumpkörper zum intraoperativen Spülkatheter, verhindert aber, dass das Spülmedium zurückgedrückt wird, insbesondere dann, wenn Spülmedium aus dem Vorratsbehälter ausläuft. Des weiteren wird durch Drücken der Schlauchklemme der Spülmediendurchfluss durch die Schlauchleitung begrenzt bzw. reguliert. Dabei ist eine mehrmalige Handbetätigung des Pumpkörpers erforderlich, um das Spülmedium zum Spülkatheter zu transportieren (Siehe auch US-A-3 398 743).

Der Erfindung liegt daher die Aufgabe zugrunde, eine Pumpe mit den gattungsgemäßen Merkmalen dahingehend zu verbessern, dass während des Spülvorganges mit einfachen Mitteln und ohne eine Aufhebung der geschlossenen Zufuhr an Spülmittel eine wirksame Erhöhung des Spüldruckes möglich ist.

Die Lösung dieser Aufgabe ergibt sich einschließlich vorteilhafter Ausgestaltungen und Weiterbildungen der Erfindung aus dem Inhalt der Patentansprüche, welche dieser Beschreibung nachgestellt sind.

Die Erfindung sieht in ihrem Grundgedanken vor, dass der Pumpkörper permanent vom Spülmedium durchströmt und das Spülmedium durch einmalige Handbetätigung zum Spülkatheter förderbar ist und in Fließrichtung des Spülmediums in einem Leitungsabschnitt der Zufuhrleitung vor dem Pumpkörper nur ein einziger, während der Handbetätigung des Pumpkörpers die Zufuhr an Spülmedium in den Pumpkörper absperrender Verschluss angeordnet ist.

Mit der Erfindung ist der Vorteil verbunden, dass keinerlei Vorbereitungshandlungen für das Aufbringen eines erhöhten Spüldrucks erforderlich sind, da der Pumpkörper von dem Spülmedium durchflossen und daher ständig in den Zufuhrbereich an Spülmedium zum Spülkatheter eingeschaltet ist; zur Erhöhung des Spüldrucks bedarf es lediglich einer manuellen Kompression des Pumpkörpers bei gleichzeitiger Absperrung der Zufuhrleitung mittels des vorgeschalteten Verschlusses. Mit dieser Handhabung wird das in dem Pumpkörper befindliche definierte Volumen an Spülmedium schlagartig in die Harnblase gepreßt, wodurch die Erhöhung des Spüldruckes bewirkt wird. Ein weiterer Vorteil der ständigen Einschaltung des Pumpkörpers in die Zufuhrleitung liegt darin, dass eine Öffnung der Zufuhrleitung für die Erhöhung des Spüldruckes nicht erforderlich ist, so dass die Infektionsgefahr auch bei Aufbringen eines erhöhten Spüldruckes nicht ansteigt.

Soweit nach einem Ausführungsbeispiel der Erfindung vorgesehen ist, dass der Pumpkörper für eine im Anschluß an seine manuelle Kompression erfolgende aktive Rückstellung in seine Ausgangsform eingerichtet ist, ergibt sich daraus der Vorteil, dass aufgrund einer eingestellten Rückstellkraft bei weiterhin gesperrtem Verschluss aufgrund der Rückstellung des Pumpkörpers in dessen Ausgangsform ein aktives Rücksaugen von Spülmedium aus der Harnblase bis in den Pumpkörper eintritt, so dass durch mehrmalige Betätigung des Pumpkörpers vorhandene Blutkoageln gelöst und auf solche Abmessungen gebracht werden können, dass ein Abgang über die Abfuhrleitung ohne weiteres erfolgt. In Ausnahmefällen sammeln sich in dem Pumpkörper Blutkoageln an, die dann im Wege einer medizinisch zweckmäßigen bzw. notwendigen Maßnahme nach einer Trennung des Pumpkörpers von der zum Spülkatheter führenden Zufuhrleitung abgespritzt und damit ebenfalls aus dem System entfernt werden können. Nach alternativen Ausführungsbeispielen kann diese Rückstellkraft entweder durch die Verwendung eines entsprechend geeigneten Materials für die Herstellung des Pumpkörpers oder aber durch Einsatz entsprechender Federn eingestellt werden, wobei diese Federn entweder innerhalb des Pumpkörpers oder auf dessen Außenfläche angeordnet sein können.

Nach Ausführungsbeispielen der Erfindung ist der Pumpkörper als manuell zusammendrückbarer Ballon ausgebildet oder alternativ als in Fließrichtung des Spülmediums kompressibler Faltenbalg.

Der in Fließrichtung des Spülmediums vor dem Pumpkörper vorgesehene Verschluß kann nach Ausführungsbeispielen der Erfindung als handbetätigte Schlauchklemme oder aber als Rückschlagventil ausgebildet sein.

In der Zeichnung sind Ausführungsbeispiele wiedergegeben, welche nachstehend beschrieben sind. Es zeigen:
- Fig. 1: in einer ausschnittsweisen Darstellung einen in die Zufuhrleitung einer Vorrichtung zum Spülen der Harnblase zwischengeschalteten Pumpkörper in einer schematischen Ansicht,
- Fig. 2: den Gegenstand der Figur 1 in einer anderen Ausführungsform.

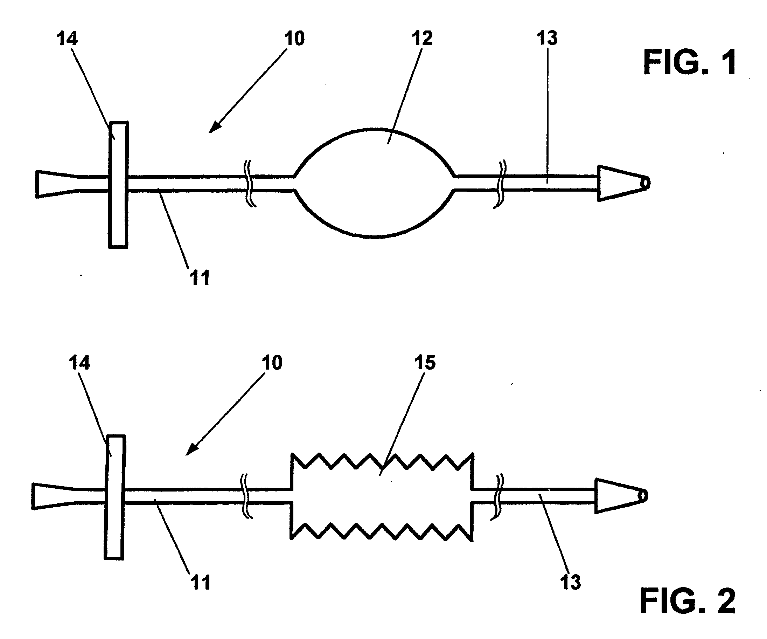

In den beiden Figuren 1 und 2 ist eine Vorrichtung zur Durchführung einer Harnblasenspülung nur ausschnittsweise dargestellt, nämlich im Bereich ihrer Zufuhrleitung, die sich zwischen einem nicht dargestellten Vorratsbehälter für das Spülmedium und einem Eingang in den Spülkatheter erstreckt. In dem Spülkatheter setzt sich die Zufuhrleitung bis in die Harnblase fort, wobei der Spülkatheter eine zweite Leitung als Abfuhrleitung für das Spülmedium aus der Harnblase aufweist.

Wie sich aus Figur 1 ergibt, ist in die Zufuhrleitung 10 ein manuell kompressibler Ballon 12 als Pumpkörper eingeschaltet, der auf der in der Ansicht linken Seite mit der Einlaufseite 11 der Zufuhrleitung 10 verbunden ist und auf der in der Zeichnungsansicht rechten Seite an einen zum nicht dargestellten Spülkatheter wegführenden Leitungsabschnitt 13 angeschlossen ist. Auf der Einlaufseite 11 ist ein Verschluß 14 angedeutet, der in einer nicht dargestellten Weise als handbetätiqte Schlauchklemme oder aber als Rückschlagventil ausgebildet sein kann.

Der Ballon 12 ist ständig vom Spülmedium durchflossen, welches von dem nicht dargestellten Vorratsbehälter über die Einlaufseite 11 der Zufuhrleitung 10, den Ballon 12 und den Leitungsabschnitt 13 zum Spülkatheter fließt. Soll der Spüldruck kurzzeitig erhöht werden, so wird der Verschluß geschlossen und es wird der Ballon 12 manuell zusammengedrückt, so daß das in dem Ballon befindliche Flüssigkeitsvolumen schlagartig in die Harnblase gedrückt wird.

In Figur 2 ist ein abgewandeltes Ausführungsbeispiel der Erfindung dargestellt, bei welchem der Ballon 12 als in Fließrichtung des Spülmediums kompressibler Faltenbalg 15 ausgebildet ist, wobei sich an diesem Faltenbalg insbesondere das alternativ vorgesehene Merkmal verwirklichen läßt, wonach der Pumpkörper für eine aktive Rückstellung in seine Ausgangsform eingerichtet sein soll. Neben den dafür vorgesehenen Maßnahmen eines geeigneten Materials oder der Anordnung einer Feder kann auch ein manuelles Auseinanderziehen des Faltenbalges 15 nach dessen Zusammendrücken erfolgen, womit der gleiche Effekt erzielbar ist.

Nach dem Zusammendrücken des Pumpkörpers sowohl in Form des Ballons 12 als auch in Form des Faltenbalges 15 ist es lediglich erforderlich, den Verschluß 14 zu öffnen, so daß sich der Pumpkörper 12, 15 wieder mit Spülmedium füllt; im Falle eines eingesetzten Rückschlagventils bedarf es keiner weiteren Maßnahme. Soll die Füllung des Ballons beschleunigt werden, so kann die Abfuhrleitung aus der Harnblase abgedrückt, d.h. gesperrt werden, so daß mangels eines Abflusses der Pumpkörper schneller wieder volläuft.

Die in der vorstehenden Beschreibung, den Patentansprüchen und der Zeichnung offenbarten Merkmale des Gegenstandes dieser Unterlagen können einzeln als auch in beliebigen Kombinationen untereinander für die Verwirklichung der Erfindung in ihren verschiedenen Ausführungsformen wesentlich sein.

## Patentansprüche

1. Vorrichtung zur Spülung der Harnblase des Menschen über einen in die Harnblase gelegten spülkatheter mit einer aus der Harnblase herausführenden Abflußleitung, bestehend aus einem ein definiertes Volumen aufweisenden kompressiblen Pumpkörper (12, 15) zur Erzeugung eines kurzzeitig erhöhten Spüldruckes zwischen einem Vorratsbehälter und dem Spülkatheter, wobei der Pumpkörper (12, 15) permanent von einem Spülmedium durchströmt und das Spülmedium durch einmalige Handbetätigung zum Spülkatheter förderbar ist, und einer an den Vorratsbehälter für das Spülmedium angeschlossenen und in die Harnblase führenden Zufuhrleitung (10), die getrennt von der Abflußleitung verläuft, **dadurch gekennzeichnet, dass** in Fließrichtung des Spülmediums in der zufuhrleitung (10) nur in der Einlaufseite (11) vor dem Pumpkörper (12, 15) ein einziger, während der Handbetätigung das Pumpkörpers (12, 15) die Zufuhr an Spülmedium in den Pumpkörper (12, 15) absperrender Verschluss (14), ohne einem weiteren Verschluss nach dem Pumpkörper (12, 15), angeordnet ist.

2. Pumpe nach Anspruch 1, **dadurch gekennzeichnet, dass** der Pumpkörper (12, 15) für eine im Anschluss an seine manuelle Kompression erfolgende aktive Rückstellung in seine Ausgangsform eingerichtet ist.

3. Pumpe nach Anspruch 2, **dadurch gekennzeichnet, dass** der Pumpkörper (12, 15) aus einem eine Rückstellkraft aufweisenden Material besteht.

4. Pumpe nach Anspruch 2, **dadurch gekennzeichnet, dass** der Pumpkörper (12, 15) mit einer die Rückstellung in seine Ausgangsform bewirkenden Feder versehen ist.

5. Pumpe nach einem der Ansprüche 1 bis 4, **dadurch gekennzeichnet, dass** der Pumpkörper als manuell zusammendrückbarer Ballon (12) ausgebildet ist.

6. Pumpe nach einem der Ansprüche 1 bis 4, **dadurch gekennzeichnet, dass** der Pumpkörper als in Fließrichtung des Spülmediums kompressibler Faltenbalg (15) ausgebildet ist.

7. Pumpe nach einem der Ansprüche 1 bis 6, **dadurch gekennzeichnet, dass** der Verschluss (14) als handbetätigte Schlauchklemme ausgebildet ist.

8. Pumpe nach einem der Ansprüche 1 bis 6, **dadurch gekennzeichnet, dass** der Verschluss (14) als Rückschlagventil ausgebildet ist.

## Claims

1. A device for flushing the human bladder by using an irrigation catheter installed into the bladder with a drainage line coming from the bladder, consisting of a compressible pump body (12,15) with a demonstrable volume, for creating a short-term increased pressure of irrigation between a supply container and the irrigation catheter, where the pump body (12, 15) is permanently filled with a flushing medium and the flushing medium can be transported with a single hand operation to the irrigation catheter and to the supply line connected to the container for the flushing medium, that leads into the bladder (10), and which runs separately from the drainage line, **characterised by** the fact that a single closure which can be shut off is fitted in the supply line (10) in the direction of flow of the flushing medium, in front of the pump body (12, 15) only on the intake side (11) and without a further closure behind the pump body (12,15), whereas the manual operation of the pump body (12, 15) supplies the flushing medium into the pump body (12,15).

2. Device according to claim 1 **characterised by** the fact that the pump element (12, 15) is designed for an active reset into its original shape following the manual compression.

3. Device according to claim 2 **characterised by** the fact that the pump element (12, 15) consists of a material with inherent restoring force.

4. Device according to claim 2 **characterised by** the fact that the pump element (12, 15) is equipped with a spring effecting the reset into its original shape.

5. Device according to one of the claims 1 to 4 **characterised by** the fact that the pump element is designed as a manually compressible balloon (12).

6. Device according to one of the claims 1 to 4 **characterised by** the fact that the pump element is designed as compressible expansion bellows (15) in the flow direction of the rinsing medium.

7. Device according to one of the claims 1 to 6 **characterised by** the fact the seal (14) is designed as a manually operated tube clamp.

8. Device according to one of the claims 1 to 6 **characterised by** the fact the seal (14) is designed as a check valve.

## Revendications

1. Dispositif destiné au rinçage de la vessie de l'homme par l'intermédiaire d'un cathéter de rinçage placé dans la vessie, avec un conduit d'évacuation sortant de la vessie, celui-ci se composant d'un corps de pompe (12,15) possédant un volume défini compressible pour la réalisation d'une pression de rinçage accrue durant une courte période, entre un réservoir de réception et le cathéter de rinçage, bien que le corps de pompe (12,15) soit traversé en permanence par un médium de rinçage et que ce médium de rinçage soit déplaçable par une activation manuelle unique sur le cathéter de rinçage, et un conduit d'amenée (10) branché au réservoir de réception et conduisant dans la vessie, le médium de rinçage qui parcourt séparément le conduit d'évacuation, **caractérisé en cela par le fait qu'**une seule fermeture obturant le passage de médium de rinçage dans le corps de pompe (12,15) est ordonnancée dans le sens de l'écoulement du médium de rinçage dans le conduit d'amenée (10), seulement sur le côté de l'entrée (11) avant le corps de pompe (12,15), pendant la manipulation du corps de pompe (12,15), sans la présence d'une autre fermeture après le corps de pompe.

2. Dispositif selon le revendication 1, **caractérisé en cela par le fait que** le corps de pompe (12,15) soit disposé pour reprendre sa forme de départ par un retour actif en position à la suite de sa compression manuelle.

3. Dispositif selon le revendication 2, **caractérisé en cela par le fait** que le corps de pompe (12,15) se compose d'un matériau doté d'une force de retour en position.

4. Dispositif selon le revendication 2, **caractérisé en cela par le fait que** le corps de pompe (12,15) est doté d'un ressort entraînant le retour en position dans sa forme de départ.

5. Dispositif selon l'une des revendications 1 à 4, **caractérisé en cela par le fait que** le corps de pompe est formé comme un ballon pliable sur lui-même (12).

6. Dispositif selon l'une des revendications 1 à 4, **caractérisé en cela par le fait que** le corps de pompe est formé comme un soufflet compressible (15) dans le sens de l'écoulement.

7. Dispositif selon l'une des revendications 1 à 6, **caractérisé en cela par le fait** que la fermeture (14) est formée comme une pince de tube commandée manuellement.

8. Dispositif selon l'une des revendications 1 à 6, **caractérisé en cela par le fait** que la fermeture (14) est formée comme une vanne anti-retour.
